(19) Europäisches Patentamt
European Patent Office
Office européen des brevets

(11) Veröffentlichungsnummer : **0 078 495**
**B1**

(12) **EUROPÄISCHE PATENTSCHRIFT**

(45) Veröffentlichungstag der Patentschrift : **28.08.85**

(51) Int. Cl.[4] : **C 07 J 19/00**, C 07 D307/58, C 07 J 9/00, C 07 J 17/00, C 07 J 43/00

(21) Anmeldenummer : **82109907.4**

(22) Anmeldetag : **27.10.82**

(54) **Verfahren zur Herstellung von Digitoxigenin.**

(30) Priorität : **30.10.81 DE 3143090**

(43) Veröffentlichungstag der Anmeldung : **11.05.83 Patentblatt 83/19**

(45) Bekanntmachung des Hinweises auf die Patenterteilung : **28.08.85 Patentblatt 85/35**

(84) Benannte Vertragsstaaten : **AT CH DE LI**

(56) Entgegenhaltungen :
**EP-A- 0 063 692**
**TETRAHEDRON LETTERS, Band 22, Nr. 35, 1981, Seiten 3385-3388, Pergamon Press Ltd., Oxford, G.B. P. WELZEL et al.: "14Beta-hydroxy - III. Synthesis of digitoxigenin from deoxycholic acid".**
**HELVETICA CHIMICA ACTA, Band 59, Fasc. 8, Nr. 295, August 1976, Seiten 2753-2764, Basel, CH. S. IWASAKI: "Photochemistry of imidazolides II. C2-C3 cleavage of carboxylic acid chains. A convenient new method for the side-chain degradation of bile acids and of lanosterol"**
**TETRAHEDRON LETTERS, Band 23, Nr. 4, 1982, Seiten 413-414, Pergamon Press Ltd., Oxford, G.B. T. MILKOVA et al.: "14Beta-hydroxy - VI. Synthesis of digitoxigenin"**

(73) Patentinhaber : **HOECHST AKTIENGESELLSCHAFT**
**Postfach 80 03 20**
**D-6230 Frankfurt am Main 80 (DE)**

(72) Erfinder : **Welzel, Peter, Prof. Dr.**
**Hustadtring 81**
**D-4630 Bochum (DE)**
Erfinder : **Stein, Herrmann**
**Hochstrasse 33**
**D-4390 Gladbeck (DE)**
Erfinder : **Milkova, Tsenka, Dr.**
**Kollegstrasse 2**
**D-4630 Bochum (DE)**

**EP 0 078 495 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der Erfindung ist ein Verfahren zur Herstellung des Digitoxigenins aus Desoxycholsäure durch Umwandlung von Desoxycholsäure (1) mit Carbonyldiimidazol in das Imidazolid (2), Abbau dieser Verbindung zur 20-Methylenverbindung (3), Konfigurationsumkehr in 3-Position und Acylierung zum 3β-Acyl-Analogen (4), Acylierung dieser Verbindung in 12-Stellung, Umsetzung des 3,12-Bis-acylats mit m-Chlorperbenzoesäure zu den Stereoisomeren 20,22-Epoxiden (6), Umlagerung der Epoxide mit einem Aluminiumalkoholat zur Allylverbindung (7), wobei die Acylgruppen verseift werden, Oxidation der Allylverbindung (7) zum α,β-ungesättigten Aldehyd (8), Umsetzung dieses Aldehyds mit Methoxymethylen-triphenylphosphoran zum Gemisch der isomeren Dienoläther (9), Umsetzung der Dienoläther (9) mit Singulett-Sauerstoff zum 1,2-Dioxan (10), Umlagerung dieser Verbindung mit einer organischen Base zum 14-α-Cardenolid (11), selektive Acetylierung in 3-Stellung und Oxidation zum 12-Keton (13), Photolyse des Ketons (13) zum ungesättigten Aldehyd (14).

Es sind bisher nur wenige Partialsynthesen auf der Grundlage von gut zugänglichen natürlichen Steroid-Vorläufern für 14β-Hydroxyl-cardenolid-aglukone bekannt geworden, die die entscheidenden für die herzwirksame Wirkung (positiv inotrope Wirkung) essentiellen Strukturmerkmale, einen 17β-konfigurierten Butenolidring sowie eine 14β-orientierte Hydroxylgruppe, enthalten. Die Schwierigkeiten und Problemstellungen der Synthese derartiger 14β-Hydroxy-cardenolid-aglukone sind von F. Sondheimer (Chem. Brit. 464 (1965)) in einzelnen aufgezählt.

Alle bisher unter diesem Gesichtspunkt erfolgreich synthetisierten 14β-Hydroxy-cardenolid-aglukone (Digitoxigenin : J ACS *84*, 875 (1962) ; Periplogenin : TL 2045 (1963) und J OC *39*, 2319 (1974) ; Xysmalogenin : Chem. Pharm. Bull., *20*, 1585 (1972) ; Uzarigenin : Liebigs Ann. Chem. *726*, 136 (1969) ; Canarigenin : ibid. *727*, 110 (1969) ; Strophanthidin : J OC *43*, 3946 (1978)) enthalten in jedem Fall die eben näher angeführten essentiellen Strukturmerkmale, eine 14β-Hydroxylgruppe sowie einen 17β-Butenolidring.

Aus Tetrahedron Letters, Band 22, 3385-3388 (1981) ist est bekannt, ein 3β-Acetoxy-12-oxo-5-β-14-α-card-22 (22)-enolid durch Photolyse in den ungesättigten Aldehyd umzuwandeln, sowie auch die übrigen Verfahrensschritte des eingangs genannten Verfahrens durchzuführen. Die ersten Verfahrensschritte sind außerdem aus Helvetica Chimica Acta *59*, 2753-2764 (1976) bekannt.

Im vorliegenden Fall wird erstmals eine neuartige und originelle Partialsynthese des Digitoxigenins auf der Ausgangsbasis Desoxycholsäure, die in 12α-Position bereits eine Hydroxylgruppe als verwertbare funktionelle Gruppe aufweist, mitgeteilt. Das Ausgangsmaterial Desoxycholsäure ist aus den natürlichen Gallensäuren leicht zugänglich.

Das erfindungsgemäße Verfahren zur Herstellung des Digitoxigenins ist dadurch gekennzeichnet, daß man bei einem Verfahren der eingangs genannten Art den ungesättigten Aldehyd mit einem metallorganischen Reduktionsmittel zum entsprechenden primären Alkohol reduziert, diesen mit einem Alkyl- oder Arylsulfonsäurehalogenid zu dem entsprechenden Sulfonsäureester umsetzt und diesen durch Solvolyse zum Digitoxigenin cyclisiert und aus diesem die Acylgruppe in 3-Stellung verseift.

Die Solvolyse von (17) zu (18) erfolgt zweckmäßig mit Oxalsäure, vorzugsweise 0,2 M Oxalsäure in Aceton-Wasser 1 : 2.

Die beschriebenen Umsetzungen sind durch die Formelbilder auf den Seiten 17, 17a und 18 veranschaulicht. In diesen Formeln und im Text bedeutet Ac Acyl, also einen Carboxylsäurerest, vorzugsweise Acetyl.

Die Photolyse von (13) ergibt durch Norrish-I-Spaltung den ungesättigten Aldehyd (14), der unter den Reaktionsbedingungen teilweise in einer intramolekularen Paterno-Büchi-Reaktion zum Oxetan (15) weiterreagiert. In schwach saurer Lösung isomerisiert das Oxetan (15) zum Aldehyd (14) zurück.

Das 3β-Acetoxy-12-oxo-5-β-14α-card-20(22)-enolid (13) kann ausgehend von der Desoxycholsäure (1) wie folgt erhalten werden : Desoxycholsäure (1) wird mit Carbonyldiimidazol in das Imidazolid (2) übergeführt und dieses nach Iwasaki photochemisch zur 20-Methylenverbindung (3) abgebaut. Aus (3) wird (4) durch Konfigurationsumkehr an $C_3$ nach dem Mitsunobu-Verfahren hergestellt. Die Acylierung von (4) ergibt (5), das mit m-Chlorperbenzoesäure zu einem 8 : 1 Gemisch der Stereoisomeren der Oxide (6) umgesetzt wird. Umlagerung der Epoxide in eine Allylalkoholgruppierung gelingt mit Aluminiumisopropylat als Base ; gleichzeitig tritt Esterhydrolyse ein. Der dabei gebildete Allylalkohol (7) wird zum ungesättigten Aldehyd (8) oxidiert, wofür insbesondere Braunstein geeignet ist, und dieser wird in einer Wittig-Reaktion mit Methoxymethylentriphenylphosphoran zum Gemisch der isomeren Dienoläther (9) umgesetzt. Die Dienoläther (9) reagieren mit Singulett-Sauerstoff in einer (4 + 2) Cycloaddition zu den 1,2-Dioxanen (10) wobei ein Gemisch der 23-Isomeren entsteht. Aus diesen wird mit einer organischen Base, z. B. Triethylamin, durch heterolytische Öffnung der Peroxidgruppierung und Lactonringschluß das 14α-Cardenolid (11) gebildet werden. Zur Einführung der 14β-OH-Gruppe wird (11) durch selektive Acylierung an $C_3$ und Oxidation and $C_{12}$ in das 12-Keton (13) umgewandelt.

Die Darstellung des 3β-Acetoxy-12α-hydroxy-5β, 14α-cardenolids (12) kann vereinfacht werden, in dem man (4) mit Singulett-Sauerstoff oxidiert, wobei ein Gemisch der 3-Acyl-Verbindungen 18 und 19 resultiert. Das darin vorhandene 18 kann abgetrennt oder, beispielsweise mit Braunstein, ebenfalls zu 19 oxidiert werden. 19 läßt sich dann, ohne daß die Zwischenstufen isoliert werden, durch Reaktion mit

Methoxymethylentriphenylhosphoran anschließender Cycloaddition mit Singulett-Sauerstoff und Umlagerung mit Triäthylamin direkt in 12 überführen.

Das gemäß der Erfindung erhaltene Verfahrensprodukt stellt aufgrund der Reaktionsführung 13 → (14) → 16 → 17 → 18, bei der Stereoselektiv die essentielle 14β-Hydroxylgruppe eingeführt wird das fertige Digitoxigenin-3-acetat dar.

Nach Verseifung der 3-Acetatgruppe zum Digitoxigenin kann dieses nach an sich bekannte Verfahren in seine in 3-Stellung glykosidierten 3-Glykoside (z. B. Rhamnoside, Glucoside, Digitoxoside etc.) überführt werden und pharmakologisch oder therapeutisch Verwendung finden.

## Beispiel 1

Abbau von Desoxycholsaäre (1) zu 20-Methylen-5β-pregnan-3α,12α-diol (3)

4.95 g (12.5 mMol) 1 (getrocknet) und 2.15 g (13 mMol) N,N'-Carbonyldiimidazol wurden in 50 ml absol. Tetrahydrofuran 3 h bei Raumtemperatur gerührt. Dann wurden nochmals 200 mg N-N'-Carbonyldiimidazol zugesetzt.

Im DC war die Bildung des polaren 2 erkennbar (DC : Aceton, 1x). Ohne weitere Aufarbeitung wurde mit absol. Tetrahydrofuran auf 500 ml verdünnt und in einer Umlaufapparatur mit fallendem Flüssigkeitsfilm (DEMA UV 13/61 mit Quarzfilter) 24 h unter Argon bei Raumteperatur mit einer Hg-Hochdrucklampe (Philips HPK-125) bestrahlt.

Anschließend wurden zur Zersetzung von noch vorhandenem N,N'-Carbonyldiimidazol 1.5 ml dest. Wasser zugesetzt und danach wurde das Lösungsmittel i.V. abgezogen.

2 derartige Photolyseansätze wurden vereinigt und an 380 g Kieselgel (Petrolether/Aceton = 4 : 1) aufgetrennt.

Erhalten wurden 3.5 g amorphes 3 (57.8 % bezogen auf umgesetztes 1) ; zurückgewonnen wurden 2.7 g (27 %) 1.

IR ($CCl_4$) : 3 600 (OH, frei) ; 3 500-3 100 (max. bei 3 450, CH, gebunden) ; 1 640 $cm^{-1}$ (C = C)

$^1$H-NMR ($CDCl_3$) : δ = 4.88, 4.77 (je ein br. s, $CH_2$-22), 3.90 (m, $W_{1/2}$ = 6 Hz, 12 β-H), 4.00-3.10 (m, 3 β-H), 3.00-2.48 (m, 17 α-H), 1.83 (s, $CH_3$-21), 1.00 (s, $CH_3$-19), 0.67 (s, $CH_3$-18)

MS : m/z (rel. Int.) = 332 (28 %), 314 (100 %), 299 (20 %), 296 (24 %), 281 (20 %).

## Beispiel 2

20-Methylen-5β-pregnan-3β,12α-diol-3-acetat (4)

1.15 g (3.5 mMol) 3, 0.21 g (3.5 mMol) Essigsäure und 1,31 g (5 mMol) Triphenylphosphin in 15 ml absol. Tetrahydrofuran wurden unter Rühren bei Raumtemperatur mit 0.87 g (5 mMol) Diethylazodicarboxylat in 5 ml Tetrahydrofuran tropfenweise versetzt. Nach Abkeingen der Wärmeentwicklung wurde 70 h bei Raumtemperatur gerührt. Abdampfen des Lösungsmittels und Auftrennung des gelben Rückstandes an 150 g Kieselgel (Petrolether (Essigester = 8 : 1) ergab 941.5 mg (73 %) 4.

Schmp. 167 °C (aus Aceton/$H_2O$)

IR ($CHCl_3$) : 3 600-3 350 (max. bei 3 450, OH), 1 720 (C = O), 1 640 (C = C), 1 260 $cm^{-1}$ (C—O)

$^1$H-NMR ($CDCl_3$) : λ = 5.05 (m, $W_{1/2}$ = 8 Hz, 3α-H), 4.85 und 4.73 (je 1 br. s, $CH_2$-22), 3.81 (m, $W_{1/2}$ = 10 Hz, 12β-H), 2.70-2.30 (m, 17α-H, 2.00 (s, 3β-OAc), 1.68 (s, $CH_3$-21), 0.93 (s, $CH_3$-19), 0.57 (s, $CH_3$-18)

MS : m/z (rel. Int.) = 374 (15 %), 356 (55 %), 341 (7 %), 314 (13 %), 296 (13 %), 285 (20 %), 281 (12 %), 134 (100 %).

## Beispiel 3

20-Methylen-5β-pregnan-3β,12α-diol-diacetat (5)

Zu 374 mg (1 mMol) 4 und 5 mg 4-Dimethylaminopyridin in 4 ml Pyridin wurden 204 mg (2 mMol) Acetanhydrid in 1 ml Pyridin bei Raumtemperatur getropft ; anschließend wurde 16 h gerührt. Pyridin und überschüssiges Anhydrid wurden weitgehend i.V. abgezogen. Der Rückstand wurde an 5 g Kieselgel (Petrolether/Essigester = 2 : 1) getrennt ; Ausbeute an 5 : 410 mg (99 %).

Schmp. 128 °C (aus Aceton/$H_2O$)

IR ($CCl_4$) : 1 730 (C = O), 1 640 (C = C), 1 240 $cm^{-1}$ (C—O)

$^1$H-NMR ($CDCl_3$) : δ = 5.00 (m, 3α-H), 4.87 (m, 12β-H), 4.82 und 4.50 (je 1 br. s, $CH_2$-22), 2.80-2.30 (s, 3β-OAc), 1.58 (br. s, $CH_3$-21), 0.93 (s, $CH_3$-19), 0.62 (s, $CH_3$-18)

MS : m/z (rel. Int.) = 416 (3 %), 374 (7 %), 356 (48 %), 341 (6 %), 328 (2 %), 313 (9 %), 312 (3 %), 296 (26 %), 281 (19 %), 134 (100 %).

## Beispiel 4

(20-R,S)-20,21 Epoxy-20-methyl-5β-pregnan-3β,12α-diol-diacetat (6)

400 mg (0.96 mMol) 5 und 250 mg (1.2 mMol) m-Chlorperbenzoesäure (85 proz.) wurden in 10 ml absol. Methylenchlorid 2 h bei Raumtemperatur gerührt.

Dann wurde einmal mit 10 ml gesättigter $Na_2S_2O_3$-Lösung und je dreimal mit je 10 ml 5 proz. $NaHCO_3$-Lösung und mit $H_2O$ gewaschen. Die wässrigen Phasen wurden einmal mit Methylenchlorid extrahiert. Die vereinigten organischen Phasen wurden über $Na_2SO_4$ getrocknet und nach Filtration eingedampft. Es wurden 412 mg (99 %) Rohprodukt und nach Umkristallisation aus Petrolether/Essigester 368 mg (89 %) der beiden Diastereomeren 6 erhalten, die chromatographisch nicht getrennt werden konnten. Nach $^1$H-NMR ergibt sich ein Diastereomerenverhältnis von 8 : 1.

Schmp. 182-185 °C (aus Aceton/$H_2O$)

IR ($CHCl_3$) : 1 720 (C = O), 1 260 und 1 240 $cm^{-1}$ (C—O)

$^1$H-NMR ($CDCl_3$) : δ = 5.00 (m, $W_{1/2}$ = 6 Hz, 3α,12β-H), 2.80 und 2.40 (je 1 m, $CH_2$-21), 2.06 (s, 12α-OAc), 2.00 (s, 3β-OAc), 1.20 (s, $CH_3$-22), 0.97 und 0.90 (je 1 s, 8 : 1, $CH_3$-19), 0.77 und 0.60 (je 1 s, 8 : 1, $CH_3$-18)

MS : m/z (rel. Int.) = 432 (15 %), 372 (100 %), 357 (41 %), 312 (22 %), 297 (17 %).

## Beispiel 5

20-Methylen-5β-pregnan-3β,12α,21-triol (7)

432 mg (1 mMol) 6 und 430 mg (2 mMol) Aluminiumtriisopropylat wurden in 25 ml absol. Toluol unter Rühren 5 h zum Sieden erhitzt. Dann wurden nochmals 430 mg Aluminiumtriisopropylat zugesetzt, und die Lösung wurde 1.5 h unter Rückfluß erhitzt. Der Ansatz wurde mit Essigester auf 100 ml verdünnt und dreimal mit je 100 ml gesättigter $(NH_4)_2SO_4$-Lösung und mit $H_2O$ gewaschen. Die wässrigen Phasen wurden einmal mit Essigester extrahiert und die vereinigten organischen Phasen über $Na_2SO_4$ getrocknet. Nach Filtration wurde eingedampft.

Ausbeute : 271.4 mg (78 %) 7.

Schmp. 167-167.5 °C (aus Essigester/Petrolether)

IR ($CHCl_3$) : δ 3 600-3 200 (max. bei 3 400, OH), 1 640 $cm^{-1}$ (C = C)

$^1$H-NMR ($CDCl_3$) : = 5.06 und 4.87 (je 1 m, $W_{1/2}$ = 4 Hz, $CH_2$-22), 4.17 (m, $W_{1/2}$ = 4 Hz, $CH_2$-21), 4.13 (m, $W_{1/2}$ = 6 Hz, 3α-H), 3.87 $W_{1/2}$ = 6 Hz, 12β-H), 3.70-3.10 (m, 17α-H), 0.95 (s. $CH_3$-19), 0.60 (s. $CH_3$-18)

MS : m/z (rel. Int.) = 348 (2 %), 330 (100 %), 315 (30 %), 312 (30 %).

## Beispiel 6

3β,12α-Dihydroxy-20-methylen-5β-pregnan-21-al (8)

140 mg (0.40 mMol) 7 und 1.0 g Mangandioxid (aktiviert) wurden in 3 ml absol. Methylenchlorid 1 h bei Raumtemperatur gerührt. Das Reaktionsgemisch wurde dann filtriert, das Filtrat i.V. eingedampft und der Rückstand an 5 g Kieselgel (Petrolether/Essigester = 3 : 1, dann 1 : 1) chromatographisch getrennt.

Ausbeute an 8 : 96.3 mg (69 %)

Schmp. 192-193 °C (aus Essigester/Petrolether)

IR ($CHCl_3$) : 3 600-3 200 (max. bei 3 480, OH) 2 710 (CHO), 1 680 (C = O, ungesättigt) 1 620 $cm^{-1}$ (C = C, konjugiert)

$^1$H-NMR ($CDCl_3$) : δ = 9.47 (s, CHO), 6.30 und 6.12 (je 1 br. s, $CH_2$-22), 4.15 (m, $W_{1/2}$ = 6 Hz, 12β-H), 4.03 (m, $W_{1/2}$ = 6 Hz, 3α-H), 3.27 (m, $W_{1/2}$ = 6 Hz, 17α-H), 0.90 (s, $CH_3$-19), 0.51 (s, $CH_3$-18)

MS : m/z (rel. Int.) = 346 (34 %), 331 (2 %), 328 (9 %), 318 (5 %), 313 (100 %).

## Beispiel 7

Umsetzung des Aldehyds 8 mit Methoxymethylentriphenylphosphoniumchlorid und Kalium-tert.-butylat

123.2 mg (0.36 mMol) Methoxymethylentriphenylphosphonium chlorid wurden unter Argon in 15 ml absol. Ether suspendiert. Bei − 25 °C wurden unter Rühren 121.2 mg (1.08 mMol) Kalium-tert-butylat zugesetzt. Die charakteristische Rotorange färbung des Ylids entstand innerhalb von 1 Min. Es wurde noch 10 Min. gerührt dann wurden bei − 25 °C 103.8 mg (0.30 mMol) 8 zugesetzt. Der Reaktionsansatz entfärbe sich allmählich (leicht gelbliche Suspension). Unter Rühren wurde dann innerhalb von 30 Min. auf Raumtemperatur aufgewärmt. Die Feststoffe wurden durch Zentrifugieren entfernt und zweimal mit Ether gewaschen. Die vereinigten organischen Phasen wurden bei 25 °C i. V. eingedampft und ergaben 101.0 mg mit Triphenylphosphinoxid verunreinigte Dienolether 9.

Die Dienolether 9 sind sehr empfindliche Substanzen, sie wurden deshalb ohne weitere Reinigung umgesetzt. Ihre Bildung wurde durch DC (2 Flecken, polarer als 8, Laufmittel : Petrolether/Essigester = 1 : 2) und spektroskopisch nachgewiesen.

CD (Cyclohexan) : $\lambda_{max}$ = 237 nm

$^1$H-NMR (Pyridin-$d_5$) : δ = 7.40-7.10 (aromat. H von $(C_6H_5)_3PO$), 5.85 (m, $W_{1/2}$ = 6 Hz, 23-H), 5.20-4,90

(m, $CH_2$-21 und 22-H), 4.30, 4.25, 4.13 und 4.00 (3α-H, 12β-H, 2x OH), 3.48 und 3.42 (je 1 s, $OCH_3$, 2 Isomere, 3.30 (s, $OCH_3$, überschuß Reagenz), 3.10 (m, $W_{1/2}$ = 5 Hz, 17α-H), 0.95 (s, $CH_3$-19), 0.65 (s, $CH_3$-18)

MS : m/z (rel. Int.) = 374 (100 %), 356 (30 %), 342 (77 %), 341 (27 %), außerdem 277 (100 %) und 201 (100 %) von $(C_6H_5)_3PO$.

## Beispiel 8

Reaktion der Dienolether 9 mit Singulett-Sauerstoff :

Eine Lösung von 25 mg (0.07 mMol) 9 enthaltenden Gemisches (s. o.) und 0.5 mg Bengalrosa B in 5 ml absol. Methylenchlorid, durch die ein trockener, mit Methylenchlorid gesättigter Sauerstoffstrom geleitet wurde, wurde bei 25 °C unter Rühren 10 Min. mit einer 1000 W-Halogenlampe bestrahlt. Durch DC (Petrolether/Essigester = 1 : 2) wurde nachgewiesen, daß die Dienolether zu einem polareren Produkt abreagiert hatten. Nach Eindampfen i. V. bei 25 °C zeigte das $^1$H-NMR-Spektrum ($CCl_4$) folgende Signale :

δ = 7.40-7,10 (aromat. H von $(C_6H_5)_3PO$), 5.45 (m, 22-H), 4.90 (m, 23-H), 4.60-3.70 (mehrere m, 3α-H, 12β-H, $CH_2$-21, 2x OH), 4.15 und 4.05 (je 1 s, $OCH_3$, 2 Isomere), 0.90 (s, $CH_3$-19), 0.60 und 0.52 (je 1 s, $CH_3$-18), 2 Isomere)

## Beispiel 9

3β,12α-Dihydroxy-5β,14ξ-card-20 (22)-enolid (11)

Zu 40 mg (0.1 mMol) eines die Dioxane 10 enthaltenden Gemisches (s. o.) in 3 ml absol. Methylenchlorid wurden unter Rühren bei Raumtemperatur 100 mg (1 mMol) Triethylamin zugesetzt, dann wurde noch 5 Min. gerührt. Laut DC (Petrolether/Essigester = 1 : 2, 1 x) hatten die Dienolether 10 vollständig zu 11 abreagiert. Der Ansatz wurde zur Trockne eingedampft. Ausbeute : 37 mg.

$^1$H-NMR ($CDCl_3$) : δ = 5.70 (m, $W_{1/2}$= 3 Hz, 22-H), 4.70 (br. s, $CH_2$-21), 4.00 (m, $W_{1/2}$ = 6 Hz 3α-H), 3.70 (m, $W_{1/2}$ = 5 Hz, 12β-H), 3.06 (m, $W_{1/2}$ = 3 Hz, 17α-H), 0.93 (s, $CH_3$-19), 0.60 (s, $CH_3$-18)

MS : m/z (rel. Int.) 374 (5 %), 356 (16 %), 346 (6 %), 341 (5 %), 338 (7 %), 328 (100 %), 313 (56 %).

## Beispiel 10

3β-Acetoxy-12α-hydroxy-5β-14α-card-20(22)-enolid (12) :

49.1 mg (0.13 mMol) 11 und 18.3 mg (0.18 mMol) Acetanhydrid wurden bei 40 °C 10 h gerührt ; nach 6 h wurden nochmals 18.5 mg (0.18 mMol) Acetanhydrid zugesetzt. Das Reaktionsgemisch wurde mit einigen Tropfen $H_2O$ versetzt und 1 Min. gerührt ; anschließend wurde bei 35 °C zur Trockne eingedampft. Der Rückstand wurde säulenchromatographisch an 5 g Kieselgel getrennt (Petrolether/Essigester = 3 : 1) und ergab 23.2 mg (63.8 %, bezogen auf umgesetztes 11) 12. Es wurden 16.4 mg (33.4 %) 11 zurückgewonnen.

Ausgehend von betrug die Ausbeute an 12 15 %.

IR ($CCl_4$) : 3 350-3 200 (max. bei 3 470, OH), 1 785 und 1 745 (Lacton), 1 740 (Acetat), 1 630 (C = C), 1 260 $cm^{-1}$ (C—O)

$^1$H-NMR ($CDCl_3$) : δ = 5.80 (m, $W_{1/2}$ = 6 Hz, 22-H), 5.00 (m, $W_{1/2}$) = 7 Hz, 3α-H), 4.73 (br. s, $CH_2$-21), 3.80 (m, $W_{1/2}$) = 8 Hz, 12β-H), 3.10 (m, $W_{1/2}$ = 8 Hz, 17α-H), 2.00 (s, 3β-OAc), 0.92 (s, $CH_3$-19), 0.62 (s, $CH_3$-18)

MS : m/z (rel. Int.) = 416 (6 %), 398 (3 %), 356 (28 %), 341 (6 %), 338 (16 %), 328 (100 %), 327 (52 %), 323 (12 %), 313 (29 %).

## Beispiel 11

3β-Acetoxy-12-oxo-5β,14α-card-20(22)-enolid (13) :

17.5 mg (0.042 mMol) 12 in 0.5 ml absol. Methylenchlorid gelöst wurden zu 13.6 mg (0.063 mMol) Pyridiniumchlorochromat und 1.8 mg (0.013 mMol) Natriumacetat-trihydrat in 0.5 ml Methylenchrlorid unter Rühren zugegeben. Nach 30 Min. wurde mit 2 ml absol. Ether versetzt und 1 h gerührt ; anschließend wurde über 0.5 g Kieselgel filtriert und mit Ether eluiert. Ausbeute: 13.2 mg (77 %) 13.

$^1$H-NMR ($CDCl_3$) δ = 5.87 (m, $W_{1/2}$ = 3 Hz, 22-H), 5.07 (m, $W_{1/2}$ = 6 Hz, 3α-H), 4.85 (br. s, $CH_2$-21) 3.03 (t, 17α-H), 2.06 (s, 3β-OAc), 1.10 (s, $CH_3$-18), 0.92 (s, $CH_3$-19)

MS : m/z (rel. Int.) = 414 (32 %), 339 (17 %), 386 (3 %), 372 (3 %), 370 (1 %), 354 (100 %), 339 (59 %), 336 (59 %), 326 (10 %), 311 (27 %).

## Beispiel 12

3β-Acetoxy-12-hydroxy-5β-12,13-seco-card-13,20)22)-dienolid (4)

20 mg 13 in 2 ml $CH_2Cl_2$ wurden unter Durchleiten von Argon bei Raumtemperatur 130 min mit einer Hg-Hochdrucklampe (Philips HPK 125) durch ein Pyrexfilter bestrahlt. Nach Eindampfen wurden die Photolyseprodukte in 0.6 ml Ethanol gelöst, bei 0 °C mit 1.0 mg $NaBH_4$ versetzt und 10 min bei dieser Temperatur gerührt. Aufgearbeitet wurde durch Neutralisieren mit 0.2 N HCl, Verdünnen mit Wasser, Extrahieren mit $CH_2Cl_2$. Säulenchromatographische Trennung (Petrolether/Essigester 1 : 1) ergab 8.5 mg (43 % bezogen auf 13) an 16.

$^1$H-NMR (80 MHz, $CDCl_3$) : δ = 0.93 (s, $CH_3$-19), 1.54 (verbreitertes s, $CH_3$-18), 1.98 (s, 3β-OAc), 3.40 (m, $CH_2$-12), 4.60 (d, $CH_2$-21), 5.00 (m, 3α-H), 5.66 (22-H).

## Beispiel 13

Digitoxigenin-3-acetat (18)

Zu 8.0 mg 16 in 200 μl absol $CH_2Cl_2$ wurden bei − 70 °C 7 μl Triethylamin und 4 μl Methansulfonylchlorid zugespritzt. Nach 2 h wurde mit $CH_2Cl_2$ verdünnt, mit Eiswasser gewaschen, getrocknet, eingedampft. Das Reaktionsprodukt wurde 1 h bei 50 °C in 1.6 ml 0.2 M oxalsäure in Aceton/Waser 1 : 2 gerührt. Dann wurde aufgearbeitet durch Verdünnen mit $CHCl_3$, Waschen mit Wasser, Trocknen. Säulenchromatographische Trennung (Petrolether/Essigester 1 : 1) ergab 2 mg (26 5) 19 und 4.2 mg (52 %, bezogen auf 16) 18, das identisch mit einer authentischen Probe war.

## Beispiel 14

20-Methylen-5β-pregnan-3β,12α,21-triol-3-acetat (20)

Die Lösung von 935 mg (2.5 m mol) 4 und 20 mg Methylenblau B in 80 ml Aceton, durch die ein trockener, mit Aceton gesättigter Sauerstoffstrom geleitet wurde, wurde unter Rühren bei 25 °C 11 h mit einer 1 000 W-Halogenlampe bestrahlt. Nach Zugabe von 1 g Natriumiodid wurde 1 h bei Raumtemperatur gerührt. Dann wurde die Lösung auf ein Drittel eingeent, mit 50 ml Essigester versetzt und je dreimal mit $Na_2S_2O_3$-Lösung und mit Wasser (jeweils 50 ml) gewaschen. Nach Trocknen über $Na_2SO_4$ und Filtration wurde i. V. eingedampft. Auftrennung des Rückstandes an 80 g Kieselgel (Petrolether/Essigester = 9 : 1) ergab 254.4 mg (36 % bezogen auf umgesetztes 4) 20 + 199.8 mg (23.5 %) 21

Wiedergewonnen wurden 258.0 mg (28 %) 4.

Schmp. : 163-164 °C (aus Aceton/Wasser)

IR ($CCl_4$) : 3 550-3 200 (OH), 1 730 (C = O), 1 240 $cm^{-1}$ (C—O)

$^1$H-NMR ($CDCl_3$) : δ = 4.97 (m, $W_{1/2}$ = 5 Hz, 3δ-H), 4.97 und 4.77 (je 1 m, $W_{1/2}$ = 5 Hz, $CH_2$-22), 4.10 (br. s, $CH_2$-21), 3.80 (m, $W_{1/2}$ = 8 Hz, 12β-H), 3.45 (m, $W_{1/2}$ = 15 Hz, 17α-H), 2.00 (s, 3β-OAc), 0.94 (s, $CH_3$-19), 0.60 (s, $CH_3$-18)

MS : m/z (rel. Int.) = 372 (33 %), 354 (14 %), 339 (2 %), 330 (7 %), 312 (38) %, 297 (15 %), 288 (13 %), 107 (100 %).

## Beispiel 15

3β-Acetoxy-12α-hydroxy-20-methylen-5β-pregnan-21-al (21)

A) aus 4 :

Bei der Darstellung von 20 wurden 199.7 mg (23.5 % bezogen auf den Umsatz an 4) 21 erhalten.

B) aus 20 :

156 mg (0.4 m mol) 20 und 1.50 g Mangandioxid (aktiviert) wurden in 5 ml absol. Methylenchlorid 30 Minuten bei Raumtemperatur gerührt. Nach Filtration wurde das Reaktionsgemisch an 8 g Kieselgel (Methylenchlorid, danach Aceton) chromatographisch getrennt.

Ausbeute an 21 : 137 mg (88 %).

C) aus 8 :

34.6 mg (0.10 m mol) 8, 12 mg (0.12 m mol) Acetanhydrid und 0.5 mg 4-Dimethylaminopyridin in 1 ml Pyridin wurden 3 h bei Raumtemperatur gerührt. Das Lösungsmittel wurde weitgehend i. V. abgezogen. Der Rückstand wurde an 1 g Kieselgel (Petrolether/Essigeter = 2 : 1) getrennt.

Ausbeute an 21 : 37.2 mg (96 %)

Schmp. : 172-174 °C (aus Petrolether/Essigester)

IR ($CCl_4$) : 3 550-3 250 (Max. bei 3 470, OH), 2 710 (Schulter, CHO), 1 720 (Ester), 1 680 (C = O, ungesättigt), 1 260 (C—O)

$^1$H-NMR ($CCl_4$) : δ = 9.40 (s, CHO), 6.27 und 6.09 (je 1 br. s, $CH_2$-22), 4.93 (m, $W_{1/2}$ = 3α-H), 3.70 (m, $W_{1/2}$ = 7 Hz, 12β-H), 3.13 (m, $W_{1/2}$ = 8 Hz, 17α-H), 1.97 (s, 3β-OAc), 0.90 (s, $CH_3$-19), 0.45 (s, $CH_3$-18)

MS : m/z (rel. Int.) = 388 (35 %), 370 (9 %), 360 (4 %), 355 (100 %), 328 (13 %), 310 (13 %), 304 (65 %).

Beispiel 16

3β-Acetoxy-12α-hydroxy-5β,14α-card-20(22)-enolid (12) aus 21

41.0 mg (0.12 m mol) Methoxymethylentriphenylphosphoniumchlorid wurden unter Argon in 5 ml absol. Ether suspendiert. Bei − 25 °C wurden unter Rühren 13.5 mg (0.12 m mol) Kalium-tert-butylat zugesetzt. Es wurde nach Entstehen des Ylids (Rotorangefärbung) noch 10 Minuten gerührt, dann wurden bei − 25 °C 37.2 mg (0.096 m mol) 21 zugesetzt. Nach Entfärbung wurde 20 Minuten gerührt, dann wurde auf Raumtemperatur aufgewärmt.

Die Feststoffe wurden durch Zentrifugieren entfernt und zweimal mit Ether gewaschen. Die Etherphasen wurden bei 25 °C i. V. eingedampft. Der Rückstand wurde zusammen mit 0.5 mg Bengalrosa B in 5 ml absol. Methylenchlorid gelöst. Durch die Lösung wurde ein trockener, mit Methylenchlorid gesättigter Sauerstoffstrom geleitet und bei 25 °C wurde unter Rühren 20 minuten mit einer 1 000 W-Halogenlampe bestrahlt. Nach Zusatz von 100 mg Triethylamin wurde 10 Minuten bei Raumtemperatur gerührt und dann zur Trockene eingedampft. Der Rückstand wurde säulenchromatographisch an 5 g Kieselgel (Petrolether/Essigester = 3 : 2) aufgetrennt.

Ausbeute an 12 : 18.5 mg (57 % bezogen auf umgesetztes 21 Wiedergewonnen wurden 7.0 mg (19 %) 21.

(Siehe Tabellen, Seite 7 ff.)

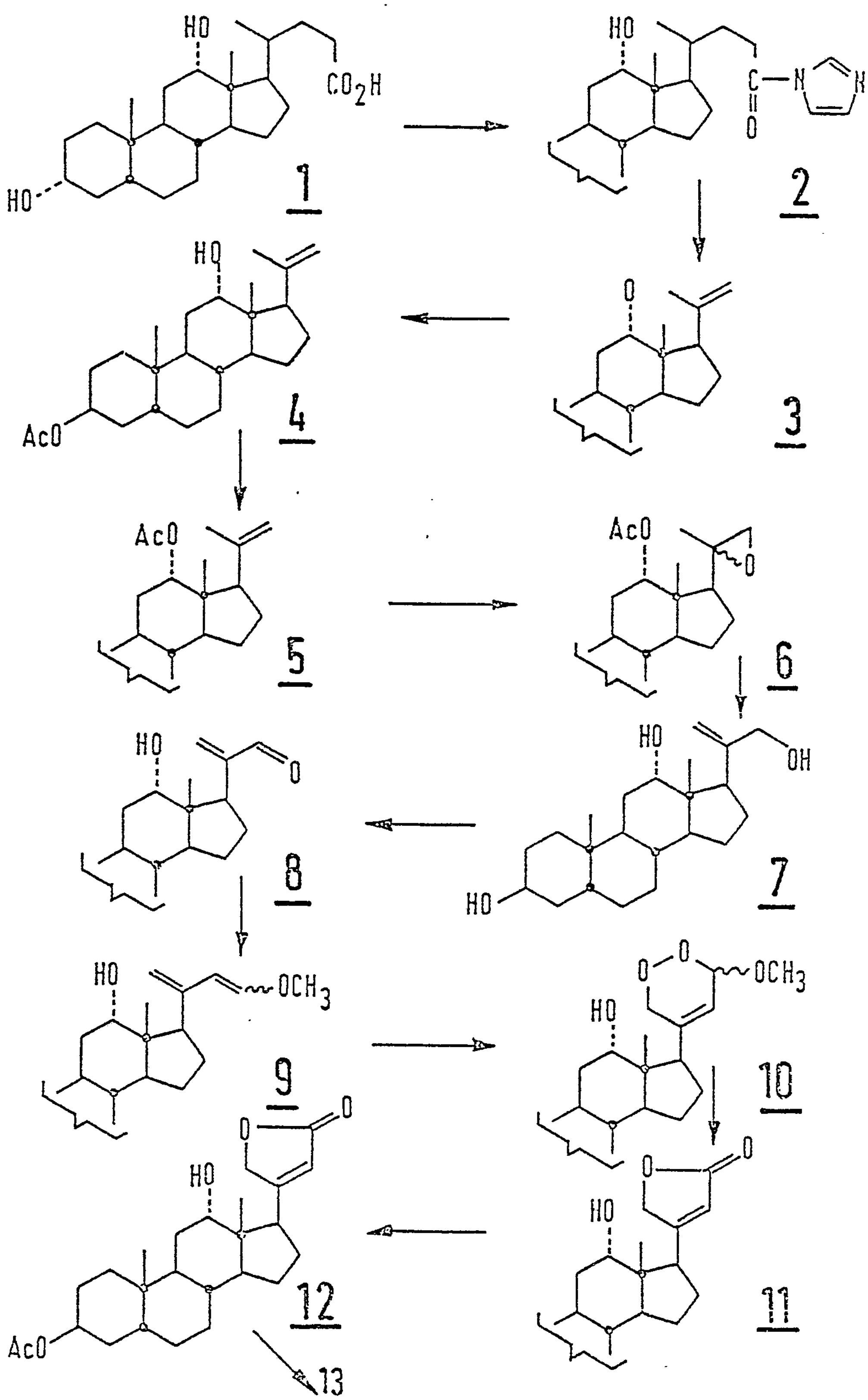

1 2 3 4 5 6 7 8 9 10 11 12 13

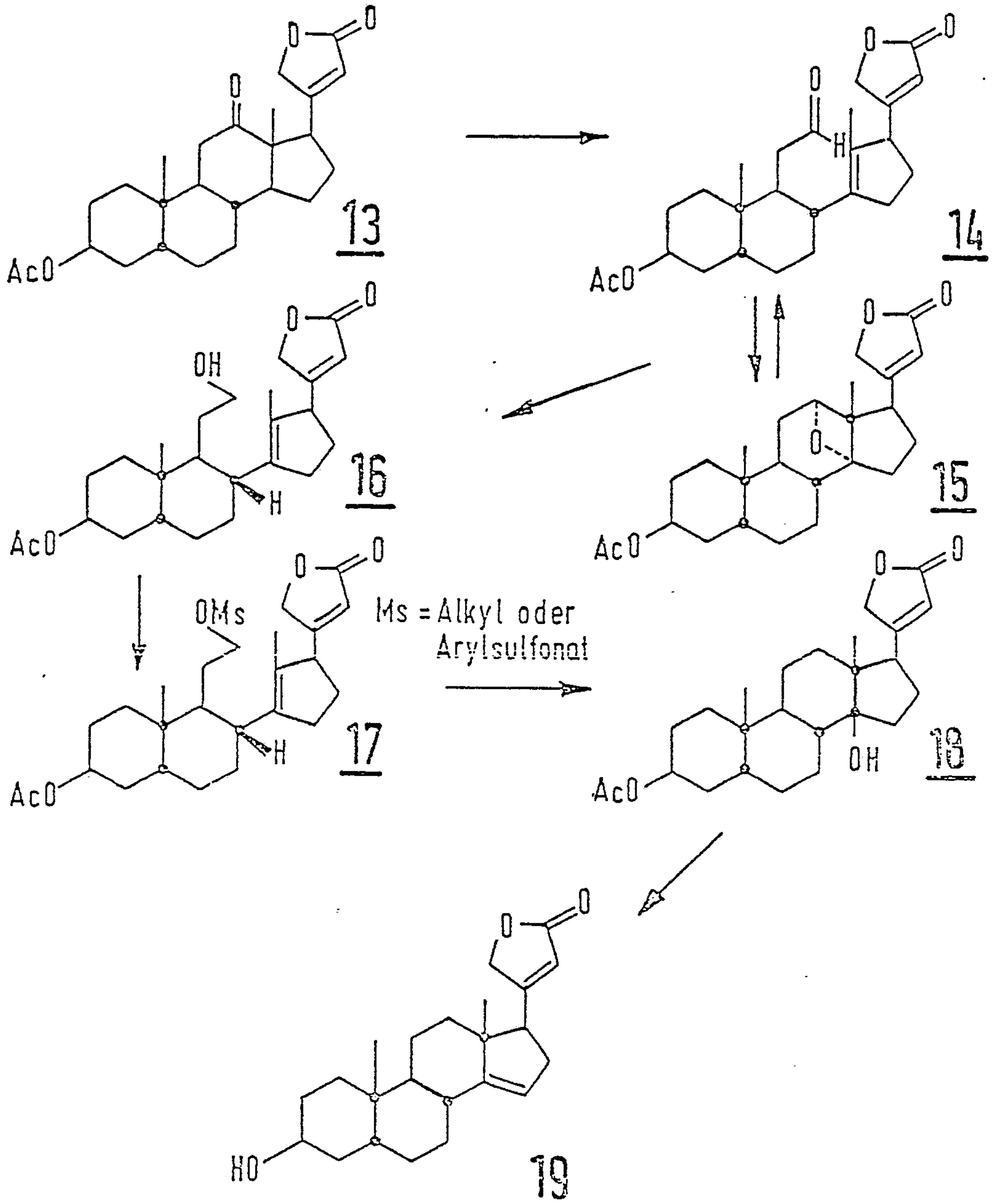


13 14 15 16 17 18 19

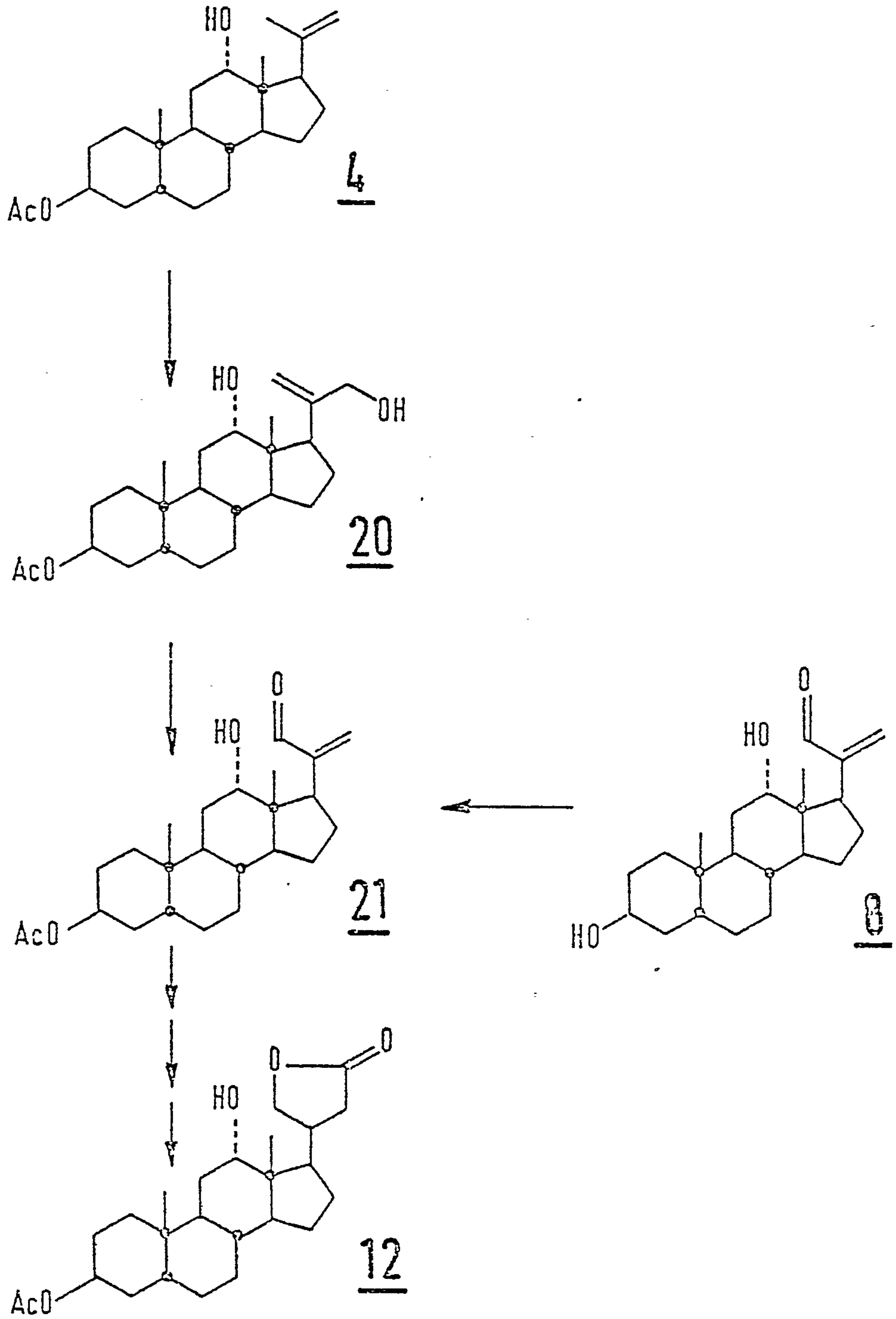

4

20

21

8

12

**Patentansprüche**

1. Verfahren zur Herstellung des Digitoxigenins aus Desoxycholsäure durch Umwandlung von
1) Desoxycholsäure (1) mit Carbonyldiimidazol in das Imidazolid (2)
2) Abbau dieser Verbindung zur 20-Methylenverbindung (3)
3) Konfigurationsumkehr in 3-Position und Acylierung zum 3β-Acyl-Analogen (4),
4) Acylierung dieser Verbindung in 12-Stellung, Umsetzung des 3,12-Bis-acylats mit m-Chlorperbenzoesäure zu den Stereoisomeren 20,22-Epoxiden (6)
5) Umsetzung der Epoxide mit einem Aluminiumalkoholat zur Allylverbindung (7), wobei die Acylgruppen verseift werden,
6) Oxidation der Allylverbindung (7) zum α,β-ungesättigten Aldehyd (8)
7) Umsetzung dieses Aldehyds mit Methoxymethylentriphenylphosphoran zum Gemisch der isomeren Dienoläther (9)
8) Umsetzung der Dienoläther (9) mit Singulett-Sauerstoff zum 1,2-Dioxan (10),
9) Umlagerung dieser Verbindung mit einer organischen Base zum 14-Cardenolid (11),
10) selektive Acetylierung in 3-Stellung und Oxidation zum 12-Keton (13),
11) Photolyse des Ketons (13) zum ungesättigten Aldehyd (14),
12) dadurch gekennzeichnet, daß man diesen mit einem metallorganischen Reduktionsmittel zum entsprechenden primären Alkohol reduziert, diesen mit einem Alkyl- oder Arylsulfonsäurehalogenid zu dem entsprechenden Sulfonsäureester umsetzt und diesen durch Solvolyse zum Digitoxigenin cyclisiert und aus diesem die Acylgruppe in 3-Stellung verseift.

2. Verfahren zur Herstellung des Digitoxigenins aus Desoxycholsäure durch Umwandlung von
1) Desoxycholsäure (1) mir Carbonyldiimidazol in das Imidazolid (2)
2) Abbau dieser Verbindung zur 20-Methylenverbindung (3)
3) Konfigurationsumkehr in 3-Position und Acylierung zum 3β-Acyl-Analogen (4),
4) Oxidation dieser Verbindung mit Singulett-Sauerstoff zum 20-Methylen-21-ol-analogen (20).
5) Oxidation der Allylverbindung (20) zum α,β-ungesättigten Aldehyd (21)
6) Umsetzung dieses Aldehyds mit Methoxymethylentriphenylphosphoran zum Gemisch der isomeren Dienoläther
7) Umsetzung der Dienoläther mit Singulett-Sauerstoff zum 1,2-Dioxan
8) Umlagerung dieser Verbindung mit einer organischen Base zum 14-Cardenolid
9) Oxidation dieser Verbindung zum 12-Keton (13)
10) Photolyse des Ketons (13) zum ungesättigten Aldehyd (14),
11) dadurch gekennzeichnet, daß man diesen mit einem metallorganischen Reduktionsmittel zum entsprechenden primären Alkohol reduziert, diesen mit einem Alkyl- oder Arylsulfonsäurehalogenid zu dem entsprechenden Sulfonsäureester umsetzt und diesen durch Solvolyse zum Digitoxigenin cyclisiert und aus diesem die Acylgruppe in 3-Stellung verseift.

3. Verbindung der Formel

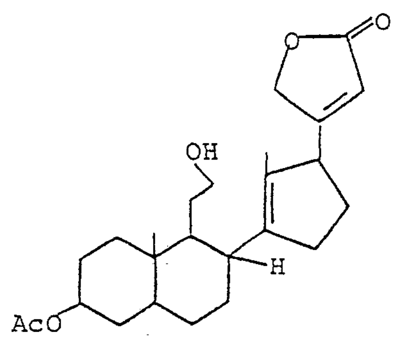

in der Ac Acyl, vorzugsweise Acetyl ist.

**Claims**

1. A process for the preparation of digitoxigenin from deoxycholic acid by conversion of
1) deoxycholic acid (1) into the imidazolide (2) with carbonyldiimidazole,
2) degradation of this compound to the 20-methylene compound (3),
3) inversion of configuration at the 3-position and acylation to give the 3β-acyl analog (4),
4) acylation of this compound in the 12-position and reaction of the 3,12-bis-acylate with m-chloroperbenzoic acid to give the stereoisomeric 20,22-epoxides (6),
5) reaction of the epoxides with an aluminium alcoholate to give the allyl compound (7), the acyl groups being hydrolyzed,

6) oxidation of the allyl compound (7) to the α,β-unsaturated aldehyde (8), .

7) reaction of this aldehyde with methoxymethylenetriphenylphosphorane to give a mixture of the isomeric dienol ethers (9),

8) reaction of the dienol ethers (9) with singlet oxygen to give the 1,2-dioxane (10),

9) rearrangement of this compound with an organic base to give the 14-cardenolide (11),

10) selective acetylation in the 3-position and oxidation to the 12-ketone (13),

11) photolysis of the ketone (13) to give the unsaturated aldehyde (14),

12) which comprises comprises (sic) reducing this aldehyde to the corresponding primary alcohol with an organometallic reducing agent, reacting this alcohol with an alkyl- or aryl-sulfonic acid halide to give the corresponding sulfonic acid ester, cyclizing this ester to digitoxigenin by solvolysis and hydrolyzing the acyl group in the 3-position from this compound.

2. A process for the preparation of digitoxigenin from deoxycholic acid by conversion of

1) deoxycholic acid (1) into the imidazolide (2) with carbonyldiimidazole,

2) degradation of this compound to the 20-methylene compound (3),

3) inversion of configuration at the 3-position and acylation to give the 3β-acyl analog (4),

4) oxidation of this compound with singlet oxygen to give the 20-methylen-21-ol analog (20),

5) oxidation of the allyl compound (20) to the α,β-unsaturated aldehyde (21),

6) reaction of this aldehyde with methoxymethylenetriphenylphosphorane to give a mixture of the isomeric dienol ethers,

7) reaction of the dienol ethers with singlet oxygen to give the 1,2-dioxane,

8) rearrangement of this compound with an organic base to give the 14-cardenolide,

9) oxidation of this compound to the 12-ketone (13),

10) photolysis of the ketone (13) to give the unsaturated aldehyde (14),

11) which comprises reducing this aldehyde to the corresponding primary alcohol with an organometallic reducing agent, reacting this alcohol with an alkyl- or aryl-sulfonic acid halide to give the corresponding sulfonic acid ester, cyclizing this ester to digitoxigenin by solvolysis and hydrolyzing the acyl group in the 3-position from this product.

3. A compound of the formula

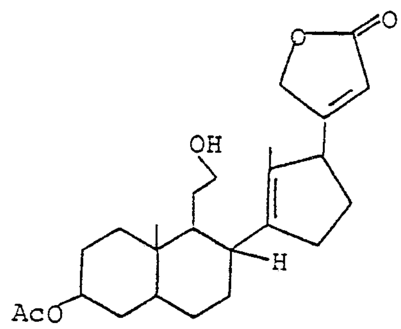

in which Ac is acyl, preferably acetyl.

**Revendications**

1. Procédé de préparation de la digitoxigénine à partir d'acide désoxycholique par transformation de

1) l'acide désoxycholique (1) avec le carbonyldiimidazole en imidazolide (2),

2) dégradation de ce composé en dérivé 20-méthylénique (3)

3) inversion de configuration en position 3 et acylation en analogue 3β-acylé (4),

4) acylation de ce composé en position 12, transformation du dérivé 3,12-bis-acylé avec l'acide m-chloroperbenzoïque en 20,22-époxydes stéréoisomères (6),

5) transformation des époxydes avec un alcoolate d'aluminium en dérivé allylique (7), les groupes acyles ayant été saponifiés,

6) oxydation du dérivé allylique (7) en aldéhyde α,β-insaturé (8),

7) transformation de cet aldéhyde avec un méthoxyméthylènetriphénylphosphoranne en un mélange de diénoléthers (9) isomères,

8) transformation des diénoléthers (9) avec de l'oxygène singulet en 1,2-dioxanne (10),

9) transposition de ce composé avec une base organique en 14-cardénolide (11),

10) acétylation sélective en position 3 et oxydation en 12-cétone (13),

11) photolyse de la cétone (13) en aldéhyde insaturée (14),

12) caractérisé en ce que l'on réduit ce dernier avec un agent réducteur organométallique en l'alcool primaire correspondant, on transforme celui-ci avec un halogénure d'acide alkyl- ou arylsulfonique en ester sulfonique correspondant et on cyclise ce dernier par solvolyse en digitoxigénine et on en élimine par saponification le groupe acyle en position 3.

2. Procédé de préparation de la digitoxigénine à partir d'acide désocycholique par transformation de
1) l'acide désoxycholique (1) avec le carbonyldiimidazole en imidazole (2),
2) dégradation de ce composé en dérivé 20-méthylénique (3),
3) inversion de configuration en position 3 et acylation en analogue 3β-acylé (4),
4) oxydation de ce composé avec de l'oxygène singulet en analogue 20-méthylène-21-ol (20),
5) oxydation du dérivé allylique (20) en aldéhyde α,β-insaturé (21),
6) transformation de cet aldéhyde avec du méthoxyméthylènetriphénylphosphoranne en un mélange de diénoléthers isomères,
7) transformation des diénoléthers avec de l'oxygène singulet en 1,2-dioxanne,
8) transposition de ce composé avec une base organique en 14-cardénolide,
9) oxydation de ce composé en 12-cétone (13),
10) photolyse de la cétone (13) en aldéhyde insaturé (14),
11) caractérisé en ce que l'on réduit ce dernier avec un agent réducteur organométallique en l'alcool primaire correspondant, on transforme celui-ci avec un halogénure d'acide alkyl- ou arylsulfonique en ester sulfonique correspondant et on cyclise ce dernier par solvolyse en digitoxigénine et on en élimine par saponification le groupe acyle en position 3.

3. Composé de formule

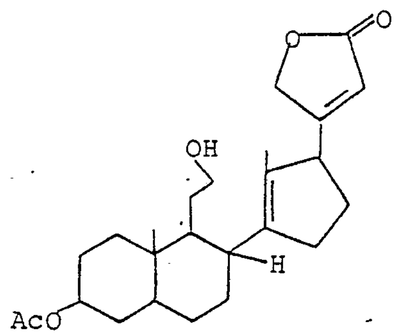

dans laquelle Ac est un acyle, en particulier acétyle.